# EUROPÄISCHE PATENTSCHRIFT

(11) **EP 4 306 054 B1**
(45) Veröffentlichungstag und Bekanntmachung des Hinweises auf die Patenterteilung: **18.11.2026**
(21) Anmeldenummer: 22217312.2
(22) Anmeldetag: 30.12.2022
(51) Int. Cl.: A61B 6/08, A61B 6/04, A61B 6/03, A61B 6/10

(54) **MEDIZINISCHES BILDGEBUNGSGERÄT UND VERFAHREN ZUM ERFASSEN VON KAMERABILDDATEN VON EINEM WECHSELWIRKUNGSBEREICH EINES MEDIZINISCHEN BILDGEBUNGSGERÄTS**
MEDICAL IMAGING DEVICE AND METHOD FOR CAPTURING CAMERA IMAGE DATA FROM AN INTERACTION AREA OF A MEDICAL IMAGING DEVICE
APPAREIL D'IMAGERIE MÉDICALE ET PROCÉDÉ DE DÉTECTION DE DONNÉES D'IMAGE DE CAMÉRA D'UNE ZONE D'INTERACTION D'UN APPAREIL D'IMAGERIE MÉDICALE

(43) Veröffentlichungstag der Anmeldung: 17.01.2024
(73) Patentinhaber: Siemens Healthineers AG, 91301 Forchheim (DE)
(72) Erfinder: Betz, Tobias, 91177 Thalmässing (DE); Ostermaier, Jochen, 91056 Erlangen (DE); Dr. Seemann, Kay Uwe, 91448 Emskirchen (DE)
(74) Vertreter: Siemens Healthineers Patent Attorneys

(56) Entgegenhaltungen:
- WO-A1-2014/120734
- WO-A1-2015/071369
- JP-A- 2019 128 206
- JP-B2- 5 371 705

## Beschreibung

Die Erfindung betrifft ein medizinisches Bildgebungsgerät.

Die Erfindung betrifft ferner ein Verfahren zum Erfassen von Kamerabilddaten von einem Wechselwirkungsbereich eines medizinischen Bildgebungsgeräts.

Während einer Bildgebungsuntersuchung kann eine Kamera verwendet werden, um das Untersuchungsobjekt zu beobachten. Elektronische Komponenten der Kamera, insbesondere der Bildsensor, können jedoch empfindlich auf Röntgenstrahlung sein. Die Röntgenstrahlung kann in der Kamera beispielsweise ein zeitveränderliches Rauschen, insbesondere in Form weißer Pixel in dem Kamerabild, einen permanenter Effekt, insbesondere in Form einer Erhöhung des Dunkelstroms, ein zufälliges Telegraphensignal (Random Telegraph Signal), eine Erhöhung der Ladungsübertragungsineffizienz (Charge Transfer Inefficiency) und/oder eine Verschlechterung der Quanteneffizienz bewirken. Die Empfindlichkeit gegenüber Röntgenstrahlung nimmt typischerweise zu, wenn die Pixel und die elektronischen Chipstrukturen der Kamera kleiner ausgeführt werden.

Als Stand der Technik sind hierbei die JP 5 371705 B2, JP 2019 128206 A, WO 2015/071369 A1 und WO 2014/120734 A1 zu nennen.

Die Erfindung hat die Aufgabe, ein Erfassen von Kamerabilddaten von einem Wechselwirkungsbereich eines medizinischen Bildgebungsgeräts mittels einer Kamera zu ermöglichen, welches in Bezug auf den Schutz der Kamera vor Röntgenstrahlen, welche aus einem Bereich eines Strahlengangs des medizinischen Bildgebungsgeräts kommen, verbessert ist.

Jeder Gegenstand eines unabhängigen Anspruchs löst diese Aufgabe. In den abhängigen Ansprüchen sind weitere vorteilhafte Aspekte der Erfindung berücksichtigt. Unabhängig vom grammatikalischen Geschlecht eines bestimmten Begriffes sind Personen mit männlicher, weiblicher oder anderer Geschlechteridentität mit umfasst.

Die Erfindung betrifft ein medizinisches Bildgebungsgerät, aufweisend
- einen Strahlengang für eine Röntgenstrahlung,
- einen Wechselwirkungsbereich, in dem ein Untersuchungsobjekt für eine Bildgebungsuntersuchung angeordnet werden kann, wobei der Strahlengang durch den Wechselwirkungsbereich hindurch verläuft,
- ein optisches System mit einem Lichteinfallsbereich und einer Kamera zum Erfassen von Kamerabilddaten von dem Wechselwirkungsbereich basierend auf Lichtstrahlen, welche aus dem Wechselwirkungsbereich kommend auf den Lichteinfallsbereich gerichtet sind und durch den Lichteinfallsbereich in das optische System einfallen, und
- ein Schutzsystem zum Schützen der Kamera vor Röntgenstrahlen, welche aus einem Bereich des Strahlengangs kommend auf den Lichteinfallsbereich gerichtet sind, wobei das Schutzsystem eine strahlungsfeste elektronische Komponente der Kamera aufweist und die strahlungsfeste elektronische Komponente der Kamera ein Bildsensor der Kamera ist.

Das medizinische Bildgebungsgerät kann insbesondere eine Strahlungsquelle und einen Strahlungsdetektor aufweisen. Die Strahlungsquelle kann insbesondere zur Emission der Röntgenstrahlung eingerichtet sein. Der Strahlungsdetektor kann insbesondere zur Detektion der Röntgenstrahlung ausgebildet sein. Der Strahlungsdetektor kann insbesondere ein quantenzählender und/oder energieauflösender Röntgendetektor sein. Der Strahlengang der Röntgenstrahlung kann sich insbesondere von der Strahlungsquelle bis zu dem Strahlungsdetektor erstrecken.

Die Röntgenstrahlung kann beispielsweise auf dem Strahlengang von der Strahlungsquelle zu dem in dem Wechselwirkungsbereich angeordneten Untersuchungsobjekt gelangen und/oder nach einer Wechselwirkung mit dem in dem Wechselwirkungsbereich angeordneten Untersuchungsobjekt zu dem Strahlungsdetektor gelangen. Bei der Wechselwirkung mit dem Untersuchungsobjekt wird die Röntgenstrahlung modifiziert und damit zum Träger von Informationen, die das Untersuchungsobjekt betreffen. Bei der Wechselwirkung der Röntgenstrahlung mit dem Strahlungsdetektor werden diese Informationen in Form von Bildgebungsdaten erfasst.

Die Lichtstrahlen, welche aus dem Wechselwirkungsbereich kommend auf den Lichteinfallsbereich gerichtet sind, können insbesondere in einem für ein menschliches Auge sichtbaren Teil des elektromagnetischen Spektrums liegen und/oder Wellenlängen aufweisen, die größer als 300 Nanometer und/oder kleiner als 800 Nanometer sind. Das medizinische Bildgebungsgerät kann beispielsweise eine Lichtquelle für die Lichtstrahlen aufweisen, beispielsweise in Form einer Beleuchtung des Wechselwirkungsbereichs, und/oder in einer beleuchteten Umgebung derart angeordnet sein, dass Licht aus der Umgebung bis zu dem Wechselwirkungsbereich gelangen kann. Die Lichtstrahlen, welche aus dem Wechselwirkungsbereich kommend auf den Lichteinfallsbereich gerichtet sind, können insbesondere Lichtstrahlen sein, die nach einer Reflexion und/oder einer Streuung an dem Untersuchungsobjekt aus dem Wechselwirkungsbereich kommend auf den Lichteinfallsbereich gerichtet sind.

Die Röntgenstrahlen, welche aus dem Bereich des Strahlengangs kommend auf den Lichteinfallsbereich gerichtet sind, können insbesondere Streustrahlen sein. Die Röntgenstrahlen, welche aus dem Bereich des Strahlengangs kommend auf den Lichteinfallsbereich gerichtet sind, können beispielsweise aus dem Wechselwirkungsbereich und/oder aus einem Bereich der Strahlungsquelle und/oder des Strahlungsdetektors kommen. Das Schutzsystem kann insbesondere zum Schützen der Kamera vor Röntgenstrahlen, welche aus dem Bereich des Strahlengangs kommend auf den Lichteinfallsbereich gerichtet sind und an dem Lichteinfallsbereich ankommen, eingerichtet sein.

Insbesondere kann vorgesehen sein, dass ein Abstand zwischen der Kamera und dem Strahlengang größer ist als ein Abstand zwischen dem Lichteinfallsbereich und dem Strahlengang.

Grundsätzlich kann ein Bauteil des medizinischen Bildgebungsgeräts gleichzeitig sowohl ein Teil des Schutzsystems als auch ein Teil des optischen Systems sein.

Das Untersuchungsobjekt kann beispielsweise ein Körperteil, insbesondere ein menschliches oder tierisches Körperteil, und/oder ein Röntgenphantom sein. Das Körperteil kann beispielsweise ein Kopf, ein Organ und/oder eine Extremität sein. Insbesondere kann eine zu untersuchende Person, beispielsweise ein Patient, das Körperteil aufweisen.

Die Kamera weist einen Bildsensor auf. Der Bildsensor der Kamera kann insbesondere zum Erzeugen der Kamerabilddaten aus den Lichtstrahlen, welche aus dem Wechselwirkungsbereich kommend auf den Lichteinfallsbereich gerichtet sind und durch den Lichteinfallsbereich in das optische System einfallen, eingerichtet sein.

Die Kamera kann beispielsweise eine 2D-Kamera und/oder eine 3D-Kamera sein. Das Funktionsprinzip der 3D-Kamera kann beispielsweise auf Stereovision, Laufzeit (Time-of-Flight), Lidar, strukturiertem Licht oder einer Kombination davon basieren. Die Kamerabilddaten können beispielsweise dazu verwendet werden, um eine Stimmung und/oder eine Bewegung einer zu untersuchenden Person zu erkennen, insbesondere automatisch zu erkennen, und/oder um eine Fernbedienung des medizinischen Bildgebungsgeräts zu unterstützen.

Es können auch mehrere Kameras, die jeweils auf die beschriebene Weise vor der Röntgenstrahlung geschützt sind, zusammen verwendet werden, beispielsweise um gleichzeitig aus verschiedenen Blickwinkeln Kamerabilddaten, insbesondere 2D-Kamerabilddaten, von dem Wechselwirkungsbereich zu erfassen.

Daraus können beispielsweise mit Hilfe eines Stereoalgorithmus kamerabasierte 3D-Bilddaten berechnet werden.

Die Kamerabilddaten können zusammen mit einer Referenzlinie, die den Kamerabilddaten überlagert ist, angezeigt werden. Die Referenzlinie kann in Form einer virtuellen Laserlinie ausgeführt sein, sodass auf echte Positionierungslaser verzichtet werden kann. Dies kann jeweils für jede von mehreren Kameras erfolgen, um eine Positionierung des Untersuchungsobjekts in Bezug auf mehrere Achsen, beispielsweise eine horizontale Achse und eine vertikale Achse, ohne Positionierungslaser zu ermöglichen.

Eine Ausführungsform sieht vor, dass das Schutzsystem ein Lichtführungssystem aufweist, welches dazu eingerichtet ist, die Lichtstrahlen, welche aus dem Wechselwirkungsbereich kommend auf den Lichteinfallsbereich gerichtet sind und durch den Lichteinfallsbereich in das optische System einfallen, weg von den Röntgenstrahlen, welche aus dem Bereich des Strahlengangs kommend auf den Lichteinfallsbereich gerichtet sind, und hin zu der Kamera zu führen, insbesondere ohne dabei die Röntgenstrahlen, welche aus dem Bereich des Strahlengangs kommend auf den Lichteinfallsbereich gerichtet sind, hin zu der Kamera zu führen.

Das Lichtführungssystem kann insbesondere ein Teil des optischen Systems sein. Mit Hilfe des Lichtführungssystems kann ein Abstand zwischen der Kamera, insbesondere dem Bildsensor der Kamera, und dem Lichteinfallsbereich vergrößert werden. Die Kamera, insbesondere der Bildsensor der Kamera, kann somit außerhalb eines durch die Röntgenstrahlung belasteten Bereichs angeordnet werden und/oder ohne Beeinträchtigung des Sichtfeldes der Kamera gegen die Röntgenstrahlung abgeschirmt werden.

Eine Ausführungsform sieht vor, dass das Lichtführungssystem einen Lichtleiter aufweist. Der Lichtleiter kann beispielsweise faseroptisch sein und/oder ein Glasfaserkabel sein. Der Lichtleiter kann insbesondere gebogen sein und/oder zum Umleiten der Lichtstrahlen eingerichtet sein.

Eine Ausführungsform sieht vor, dass das Lichtführungssystem eine Lichtablenkeinheit aufweist. Die Lichtablenkeinheit kann beispielsweise ein Spiegel und/oder ein Prisma sein. Die Lichtablenkeinheit kann insbesondere zum Ablenken der Lichtstrahlen eingerichtet sein. Das Führen der Lichtstrahlen mittels des Lichtführungssystems kann insbesondere ein Umleiten der Lichtstrahlen mittels des Lichtleiters und/oder ein Ablenken der Lichtstrahlen mittels der Lichtablenkeinheit umfassen.

Eine Ausführungsform sieht vor, dass das Lichtführungssystem endoskopisch und/oder periskopisch ist. Das Lichtführungssystem kann beispielsweise in Form eines Periskops und/oder Endoskops, insbesondere in Form eines Boroskops, ausgebildet sein.

Eine Ausführungsform sieht vor, dass das Schutzsystem eine Strahlenschutzkomponente aufweist, welche für die Lichtstrahlen, welche aus dem Wechselwirkungsbereich kommend auf den Lichteinfallsbereich gerichtet sind und durch den Lichteinfallsbereich in das optische System einfallen, transparent ist und die Kamera gegen die Röntgenstrahlung abschirmt. Die Strahlenschutzkomponente kann beispielsweise in die Kamera, den Lichteinfallsbereich und/oder in das Lichtführungssystem integriert sein.

Eine Ausführungsform sieht vor, dass die Strahlenschutzkomponente aus Strahlenschutzglas hergestellt ist. Das Strahlenschutzglas kann beispielsweise Bleiglas sein oder bleifrei sein. Die Strahlenschutzkomponente kann beispielsweise ein Eintrittsfenster der Kamera und/oder ein Eintrittsfenster des Lichteinfallsbereichs sein.

Eine Ausführungsform sieht vor, dass die Strahlenschutzkomponente eine Linse des optischen Systems ist, insbesondere eine Linse der Kamera ist.

Die Erfindung sieht vor, dass das Schutzsystem eine strahlungsfeste elektronische Komponente der Kamera aufweist, wobei die strahlungsfeste elektronische Komponente der Kamera ein Bildsensor der Kamera ist.

Eine Ausführungsform sieht vor, dass das medizinische Bildgebungsgerät eine Gantry mit einer Öffnung aufweist, wobei das Untersuchungsobjekt für die Bildgebungsuntersuchung in die Öffnung eingeführt werden kann, wobei sich der Wechselwirkungsbereich in der Öffnung befindet, wobei der Lichteinfallsbereich in eine die Öffnung begrenzende Wandung der Gantry integriert ist.

Die Öffnung kann beispielsweise tunnelförmig sein. Insbesondere bei einer tunnelförmigen Öffnung ist die direkte Sicht auf das darin angeordnete Untersuchungsobjekt von einer Bedienkonsole aus und/oder aus einem Kontrollraum heraus stark eingeschränkt. Auch das Erfassen von Kamerabilddaten basierend auf Lichtstrahlen, die aus der tunnelförmigen Öffnung heraus gelangen, ist typischerweise mit Einschränkungen in Bezug auf das Sichtfeld und/oder den Blickwinkel verbunden.

Die die Öffnung begrenzende Wandung der Gantry kann beispielsweise durch eine Verkleidung der Gantry gebildet sein. Die Verkleidung der Gantry kann insbesondere dazu eingerichtet sein, einen Innenbereich der Gantry gegen eine Umgebung abzugrenzen. Der Lichteinfallsbereich kann insbesondere an den Strahlengang der Röntgenstrahlung angrenzend angeordnet sein. Somit kann einerseits vermieden werden, dass der Strahlengang der Röntgenstrahlung durch eine Komponente des optischen Systems gestört wird. Andererseits kann somit ein möglichst großer Blickwinkel auf den Wechselwirkungsbereich realisiert werden.

Das medizinische Bildgebungsgerät kann beispielsweise ein Röntgengerät, ein Mammographiegerät, ein C-Bogen-Röntgengerät oder ein Computertomographiegerät (CT-Gerät) sein.

Das Computertomographiegerät kann beispielsweise eine Gantry mit einem Rotor, einer Drehlagerung und einer Tragstruktur aufweisen, wobei der Rotor mittels der Drehlagerung an der Tragstruktur relativ zu der Tragstruktur um eine Drehachse drehbar gelagert ist. Der Rotor kann insbesondere die Strahlungsquelle und den Strahlungsdetektor aufweisen. Das optische System kann beispielsweise an der Tragstruktur oder an dem Rotor angeordnet sein. Der Lichteinfallsbereich kann beispielsweise in Bezug auf die Drehachse zwischen der Kamera und dem Strahlengang angeordnet sein.

Die Erfindung betrifft ferner ein Verfahren zum Erfassen von Kamerabilddaten von einem Wechselwirkungsbereich eines medizinischen Bildgebungsgeräts, wobei das medizinische Bildgebungsgerät einen Strahlengang für eine Röntgenstrahlung aufweist, wobei der Strahlengang durch den Wechselwirkungsbereich hindurch verläuft, wobei ein Untersuchungsobjekt für eine Bildgebungsuntersuchung in dem Wechselwirkungsbereich angeordnet werden kann, das Verfahren umfassend
- ein Erfassen der Kamerabilddaten von dem Wechselwirkungsbereich des medizinischen Bildgebungsgeräts mittels einer Kamera eines optischen Systems basierend auf Lichtstrahlen, welche aus dem Wechselwirkungsbereich kommend auf einen Lichteinfallsbereich des optischen Systems gerichtet sind und durch den Lichteinfallsbereich in das optische System einfallen, und
- ein Schützen der Kamera vor Röntgenstrahlen, welche aus einem Bereich des Strahlengangs kommend auf den Lichteinfallsbereich gerichtet sind, wobei das Schutzsystem eine strahlungsfeste elektronische Komponente der Kamera aufweist und die strahlungsfeste elektronische Komponente der Kamera ein Bildsensor der Kamera ist.

Das erfindungsgemäße Verfahren kann insbesondere zum Erfassen von Kamerabilddaten von dem Wechselwirkungsbereich der erfindungsgemäßen medizinischen Bildgebungsgeräts verwendet werden. Das Schützen der Kamera kann insbesondere vor Röntgenstrahlen erfolgen, welche aus dem Bereich des Strahlengangs kommend auf den Lichteinfallsbereich gerichtet sind und an dem Lichteinfallsbereich ankommen. Das Schützen der Kamera kann insbesondere ein Schützen des Bildsensors der Kamera vor den Röntgenstrahlen, welche aus dem Bereich des Strahlengangs kommend auf den Lichteinfallsbereich gerichtet sind, umfassen.

Eine Ausführungsform sieht vor, dass die Lichtstrahlen, welche aus dem Wechselwirkungsbereich kommend auf den Lichteinfallsbereich gerichtet sind und durch den Lichteinfallsbereich in das optische System einfallen, mittels eines Lichtführungssystems weg von den Röntgenstrahlen, welche aus dem Bereich des Strahlengangs kommend auf den Lichteinfallsbereich gerichtet sind, und hin zu der Kamera geführt werden.

Eine Ausführungsform sieht vor, dass die Lichtstrahlen, welche aus dem Wechselwirkungsbereich kommend auf den Lichteinfallsbereich gerichtet sind und durch den Lichteinfallsbereich in das optische System einfallen, mittels eines Lichtführungssystems weg von den Röntgenstrahlen, welche aus dem Bereich des Strahlengangs kommend auf den Lichteinfallsbereich gerichtet sind, und hin zu der Kamera geführt werden, indem sie mittels eines Lichtleiters des Lichtführungssystems umgeleitet werden.

Eine Ausführungsform sieht vor, dass die Lichtstrahlen, welche aus dem Wechselwirkungsbereich kommend auf den Lichteinfallsbereich gerichtet sind und durch den Lichteinfallsbereich in das optische System einfallen, mittels eines Lichtführungssystems weg von den Röntgenstrahlen, welche aus dem Bereich des Strahlengangs kommend auf den Lichteinfallsbereich gerichtet sind, und hin zu der Kamera geführt werden, indem sie mittels einer Lichtablenkeinheit des Lichtführungssystems abgelenkt werden.

Eine Ausführungsform sieht vor, dass die Kamera mittels einer Strahlenschutzkomponente des Schutzsystems gegen die Röntgenstrahlung abgeschirmt wird, welche für die Lichtstrahlen, welche aus dem Wechselwirkungsbereich kommend auf den Lichteinfallsbereich gerichtet sind und durch den Lichteinfallsbereich in das optische System einfallen, transparent ist, und dass die Lichtstrahlen, welche aus dem Wechselwirkungsbereich kommend auf den Lichteinfallsbereich gerichtet sind und durch den Lichteinfallsbereich in das optische System einfallen, durch die Strahlenschutzkomponente des Schutzsystems hindurchgeführt werden, insbesondere mittels des Lichtführungssystems hindurchgeführt werden.

Die erfindungsgemäße Lösung ermöglicht insbesondere, Kamerabilddaten von dem Wechselwirkungsbereich des medizinischen Bildgebungsgeräts aufzunehmen, welche im Vergleich zu auf herkömmliche Weise erfassten Kamerabilddaten in Bezug auf die Bildqualität, das Sichtfeld und/oder den Blickwinkel verbessert sind.

Im Rahmen der Erfindung können Merkmale, welche in Bezug auf unterschiedliche Ausführungsformen der Erfindung und/oder unterschiedliche Anspruchskategorien (Verfahren, Verwendung, Vorrichtung, System, Anordnung usw.) beschrieben sind, zu weiteren Ausführungsformen der Erfindung kombiniert werden. Beispielsweise kann ein Anspruch, der eine Vorrichtung betrifft, auch mit Merkmalen, die im Zusammenhang mit einem Verfahren beschrieben oder beansprucht sind, weitergebildet werden und umgekehrt. Funktionale Merkmale eines Verfahrens können dabei durch entsprechend ausgebildete gegenständliche Komponenten ausgeführt werden. Die Verwendung des unbestimmten Artikels "ein" bzw. "eine" schließt nicht aus, dass das betroffene Merkmal auch mehrfach vorhanden sein kann. Der Ausdruck "basierend auf" kann im Kontext der vorliegenden Anmeldung insbesondere im Sinne des Ausdrucks "unter Verwendung von" verstanden werden. Insbesondere schließt eine Formulierung, der zufolge ein erstes Merkmal basierend auf einem zweiten Merkmal berechnet (alternativ: ermittelt, generiert etc.) wird, nicht aus, dass das erste Merkmal ferner basierend auf einem dritten Merkmal berechnet (alternativ: ermittelt, generiert etc.) werden kann.

Im Folgenden wird die Erfindung anhand von Ausführungsbeispielen unter Hinweis auf die beigefügten Figuren erläutert. Die Darstellung in den Figuren ist schematisch, stark vereinfacht und nicht zwingend maßstabsgetreu.

Die Fig. 1 zeigt ein medizinisches Bildgebungsgerät.

Die Fig. 2 zeigt ein optisches System mit einem Schutzsystem gemäß einem ersten Beispiel.

Die Fig. 3 zeigt ein optisches System mit einem Schutzsystem gemäß einem zweiten Beispiel.

Die Fig. 4 zeigt ein Ablaufdiagramm eines Verfahrens zum Erfassen von Kamerabilddaten von einem Wechselwirkungsbereich eines medizinischen Bildgebungsgeräts.

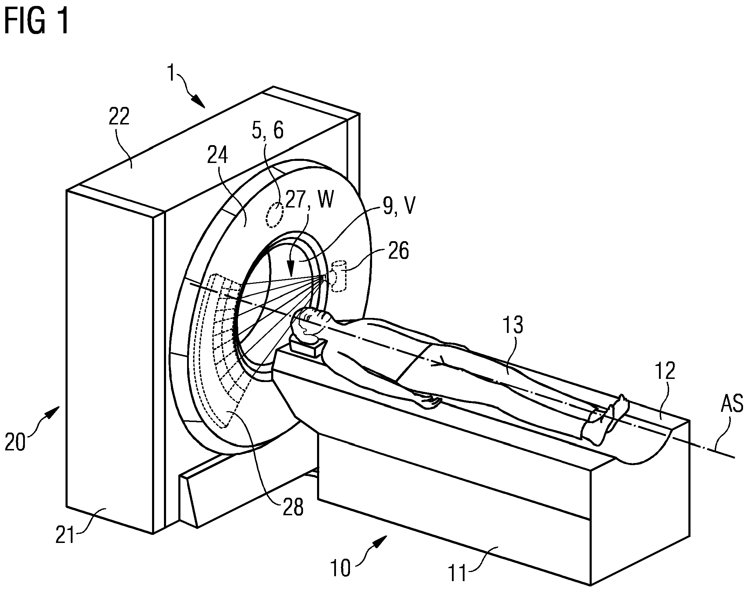

Die Fig. 1 zeigt das medizinische Bildgebungsgerät 1 in Form eines Computertomographiegeräts. Das Computertomographiegerät weist eine Gantry 20 mit einem Rotor 24, einer Drehlagerung und einer Tragstruktur aufweisen, wobei der Rotor 24 mittels der Drehlagerung an der Tragstruktur relativ zu der Tragstruktur um die Drehachse AS drehbar gelagert ist. Die Tragstruktur weist den Tragrahmen 21 und den relativ zu dem Tragrahmen 21 kippbar gelagerten Kipprahmen 22 auf. Der Rotor 24 weist die Strahlungsquelle 26 und den Strahlungsdetektor 28 auf. Das Computertomographiegerät weist eine Patientenlagerungsvorrichtung 10 zur Lagerung des Patienten 13 auf. Die Lagerungsplatte 12 kann relativ zum Lagerungssockel 11 verschoben werden, um den auf der Lagerungsplatte 12 ruhenden Patienten 13 in die Öffnung 9 einzuführen.

Das medizinische Bildgebungsgerät 1 weist den Strahlengang 27 für die Röntgenstrahlung und den Wechselwirkungsbereich W, in dem das Untersuchungsobjekt 14 für eine Bildgebungsuntersuchung angeordnet werden kann, auf, wobei der Strahlengang 27 durch den Wechselwirkungsbereich W hindurch verläuft.

Das medizinische Bildgebungsgerät 1 weist das optische System 5 mit dem Lichteinfallsbereich 51 und der Kamera 50 zum Erfassen von Kamerabilddaten von dem Wechselwirkungsbereich W basierend auf Lichtstrahlen 8, welche aus dem Wechselwirkungsbereich W kommend auf den Lichteinfallsbereich 51 gerichtet sind und durch den Lichteinfallsbereich 51 in das optische System 5 einfallen, und das Schutzsystem 6 zum Schützen der Kamera 50 vor Röntgenstrahlen 7, welche aus einem Bereich des Strahlengangs 27 kommend auf den Lichteinfallsbereich 51 gerichtet sind, auf.

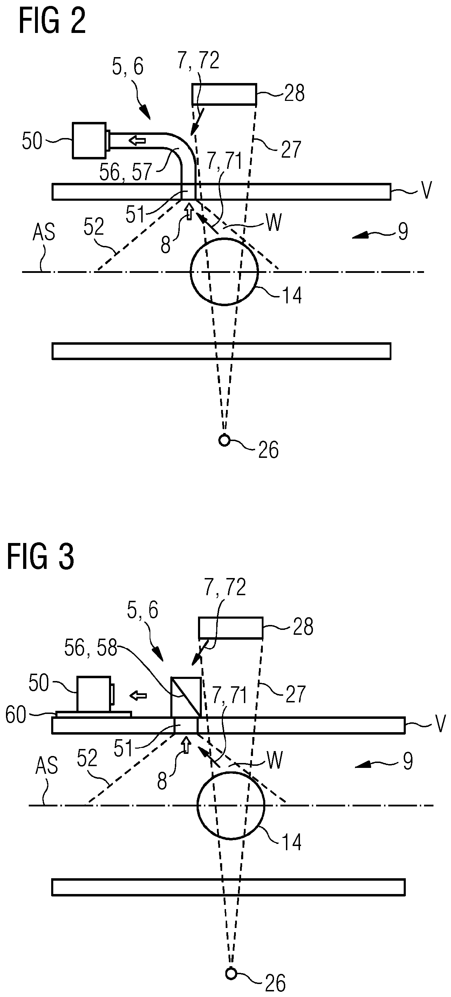

Die Fig. 2 zeigt das optische System 5 mit dem Schutzsystem 6 gemäß einem ersten Beispiel. Das Untersuchungsobjekt 14 ist beispielsweise ein Körperteil des Patienten 13. Das medizinische Bildgebungsgerät 1 weist die Gantry 20 mit der Öffnung 9 auf, wobei das Untersuchungsobjekt 14 für die Bildgebungsuntersuchung in die Öffnung 9 eingeführt werden kann, wobei sich der Wechselwirkungsbereich W in der Öffnung 9 befindet, wobei der Lichteinfallsbereich 51 in eine die Öffnung 9 begrenzende Wandung V der Gantry 20 integriert ist. Das Untersuchungsobjekt 14 befindet sich gleichzeitig sowohl in dem Wechselwirkungsbereich W des medizinischen Bildgebungsgeräts 1 als auch in dem Sichtfeld 52 der Kamera 50.

Bei den Röntgenstrahlen 7, welche aus dem Bereich des Strahlengangs 27 kommend auf den Lichteinfallsbereich 51 gerichtet sind, handelt es sich um Streustrahlen 71, welche aus dem Wechselwirkungsbereich W kommen, und Streustrahlen 72, welche aus einem Bereich der Strahlungsquelle 26 und/oder des Strahlungsdetektors 28 kommen. Der Lichteinfallsbereich 51 ist in Bezug auf die Drehachse AS zwischen der Kamera 50 und dem Strahlengang 27 angeordnet, sodass der Abstand zwischen der Kamera 50 und dem Strahlengang 27 größer ist als der Abstand zwischen dem Lichteinfallsbereich 51 und dem Strahlengang 27.

Das Schutzsystem 6 weist das Lichtführungssystem 56 auf, welches dazu eingerichtet ist, die Lichtstrahlen 8, welche aus dem Wechselwirkungsbereich W kommend auf den Lichteinfallsbereich 51 gerichtet sind und durch den Lichteinfallsbereich 51 in das optische System 5 einfallen, weg von den Röntgenstrahlen 7, welche aus dem Bereich des Strahlengangs 27 kommend auf den Lichteinfallsbereich 51 gerichtet sind, und hin zu der Kamera 50 zu führen. In diesem Beispiel weist das Lichtführungssystem 56 einen Lichtleiter 57 auf.

Die Fig. 3 zeigt das optische System 5 mit dem Schutzsystem 6 gemäß einem zweiten Beispiel, wobei das Lichtführungssystem 56 die Lichtablenkeinheit 58 aufweist. In diesem Beispiel ist die Kamera 50 zusätzlich mit der Strahlenschutzabschirmung 60 gegen die Röntgenstrahlung abgeschirmt.

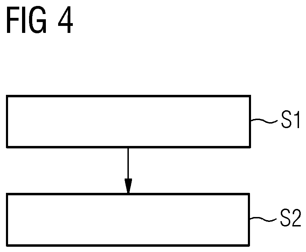

Die Fig. 4 zeigt ein Ablaufdiagramm eines Verfahrens zum Erfassen von Kamerabilddaten von dem Wechselwirkungsbereich W des medizinischen Bildgebungsgeräts 1, wobei das medizinische Bildgebungsgerät 1 den Strahlengang 27 für die Röntgenstrahlung aufweist, wobei der Strahlengang 27 durch den Wechselwirkungsbereich W hindurch verläuft, wobei der Untersuchungsobjekt 14 für die Bildgebungsuntersuchung in dem Wechselwirkungsbereich W angeordnet werden kann, das Verfahren umfassend
- das Erfassen S1 der Kamerabilddaten von dem Wechselwirkungsbereich W des medizinischen Bildgebungsgeräts 1 mittels einer Kamera 50 eines optischen Systems 5 basierend auf Lichtstrahlen 8, welche aus dem Wechselwirkungsbereich W kommend auf einen Lichteinfallsbereich 51 des optischen Systems 5 gerichtet sind und durch den Lichteinfallsbereich 51 in das optische System 5 einfallen, und
- das Schützen S2 der Kamera 50 vor Röntgenstrahlen 7, welche aus einem Bereich des Strahlengangs 27 kommend auf den Lichteinfallsbereich 51 gerichtet sind.

## Patentansprüche

1. Medizinisches Bildgebungsgerät (1), aufweisend
- einen Strahlengang (27) für eine Röntgenstrahlung,
- einen Wechselwirkungsbereich (W), in dem ein Untersuchungsobjekt (14) für eine Bildgebungsuntersuchung angeordnet werden kann, wobei der Strahlengang (27) durch den Wechselwirkungsbereich (W) hindurch verläuft,
- ein optisches System (5) mit einem Lichteinfallsbereich (51) und einer Kamera (50) zum Erfassen von Kamerabilddaten von dem Wechselwirkungsbereich (W) basierend auf Lichtstrahlen (8), welche aus dem Wechselwirkungsbereich (W) kommend auf den Lichteinfallsbereich (51) gerichtet sind und durch den Lichteinfallsbereich (51) in das optische System (5) einfallen, und
- ein Schutzsystem (6) zum Schützen der Kamera (50) vor Röntgenstrahlen (7), welche aus einem Bereich des Strahlengangs (27) kommend auf den Lichteinfallsbereich (51) gerichtet sind,
- **dadurch gekennzeichnet, dass** das Schutzsystem (6) eine strahlungsfeste elektronische Komponente der Kamera (50) aufweist, wobei die strahlungsfeste elektronische Komponente der Kamera (50) ein Bildsensor der Kamera (50) ist.

2. Medizinisches Bildgebungsgerät (1) nach Anspruch 1,
- wobei das Schutzsystem (6) ein Lichtführungssystem (56) aufweist, welches dazu eingerichtet ist, die Lichtstrahlen (8), welche aus dem Wechselwirkungsbereich (W) kommend auf den Lichteinfallsbereich (51) gerichtet sind und durch den Lichteinfallsbereich (51) in das optische System (5) einfallen, weg von den Röntgenstrahlen (7), welche aus dem Bereich des Strahlengangs (27) kommend auf den Lichteinfallsbereich (51) gerichtet sind, und hin zu der Kamera (50) zu führen.

3. Medizinisches Bildgebungsgerät (1) nach Anspruch 2,
- wobei das Lichtführungssystem (56) einen Lichtleiter (57) aufweist.

4. Medizinisches Bildgebungsgerät (1) nach Anspruch 2 oder 3,
- wobei das Lichtführungssystem (56) eine Lichtablenkeinheit (58) aufweist.

5. Medizinisches Bildgebungsgerät (1) nach einem der Ansprüche 2 bis 4,
- wobei das Lichtführungssystem (56) endoskopisch und/oder periskopisch ist.

6. Medizinisches Bildgebungsgerät (1) nach einem der Ansprüche 1 bis 5,
- wobei das Schutzsystem (6) eine Strahlenschutzkomponente aufweist, welche für die Lichtstrahlen (8), welche aus dem Wechselwirkungsbereich (W) kommend auf den Lichteinfallsbereich (51) gerichtet sind und durch den Lichteinfallsbereich (51) in das optische System (5) einfallen, transparent ist und die Kamera (50) gegen die Röntgenstrahlung abschirmt.

7. Medizinisches Bildgebungsgerät (1) nach Anspruch 6,
- wobei die Strahlenschutzkomponente aus Strahlenschutzglas hergestellt ist.

8. Medizinisches Bildgebungsgerät (1) nach Anspruch 6 oder 7,
- wobei die Strahlenschutzkomponente eine Linse des optischen Systems (5) ist.

9. Medizinisches Bildgebungsgerät (1) nach einem der Ansprüche 1 bis 8,
- wobei das Schutzsystem (6) eine strahlungsfeste elektronische Komponente der Kamera (50) aufweist.

10. Medizinisches Bildgebungsgerät (1) nach einem der Ansprüche 1 bis 9,
- wobei das medizinische Bildgebungsgerät (1) eine Gantry (20) mit einer Öffnung (9) aufweist,
- wobei das Untersuchungsobjekt (14) für die Bildgebungsuntersuchung in die Öffnung (9) eingeführt werden kann,
- wobei sich der Wechselwirkungsbereich (W) in der Öffnung (9) befindet,
- wobei der Lichteinfallsbereich (51) in eine die Öffnung (9) begrenzende Wandung (V) der Gantry (20) integriert ist.

11. Verfahren zum Erfassen von Kamerabilddaten von einem Wechselwirkungsbereich (W) eines medizinischen Bildgebungsgeräts (1), wobei das medizinische Bildgebungsgerät (1) einen Strahlengang (27) für eine Röntgenstrahlung aufweist, wobei der Strahlengang (27) durch den Wechselwirkungsbereich (W) hindurch verläuft, wobei ein Untersuchungsobjekt (14) für eine Bildgebungsuntersuchung in dem Wechselwirkungsbereich (W) angeordnet werden kann, das Verfahren umfassend
- ein Erfassen (S1) der Kamerabilddaten von dem Wechselwirkungsbereich (W) des medizinischen Bildgebungsgeräts (1) mittels einer Kamera (50) eines optischen Systems (5) basierend auf Lichtstrahlen (8), welche aus dem Wechselwirkungsbereich (W) kommend auf einen Lichteinfallsbereich (51) des optischen Systems (5) gerichtet sind und durch den Lichteinfallsbereich (51) in das optische System (5) einfallen, und
- ein Schützen (S2) der Kamera (50) vor Röntgenstrahlen (7), welche aus einem Bereich des Strahlengangs (27) kommend auf den Lichteinfallsbereich (51) gerichtet sind, wobei das medizinische Bildgebungsgerät (1) ein Schutzsystem (6) zum Schützen der Kamera (50) vor den Röntgenstrahlen (7), welche aus dem Bereich des Strahlengangs (27) kommend auf den Lichteinfallsbereich (51) gerichtet sind, aufweist,
- **dadurch gekennzeichnet, dass** das Schutzsystem (6) eine strahlungsfeste elektronische Komponente der Kamera (50) aufweist, wobei die strahlungsfeste elektronische Komponente der Kamera (50) ein Bildsensor der Kamera (50) ist.

12. Verfahren nach Anspruch 11,
- wobei die Lichtstrahlen (8), welche aus dem Wechselwirkungsbereich (W) kommend auf den Lichteinfallsbereich (51) gerichtet sind und durch den Lichteinfallsbereich (51) in das optische System (5) einfallen, mittels eines Lichtführungssystems (56) weg von den Röntgenstrahlen (7), welche aus dem Bereich des Strahlengangs (27) kommend auf den Lichteinfallsbereich (51) gerichtet sind, und hin zu der Kamera (50) geführt werden.

13. Verfahren nach Anspruch 12,
- wobei die Lichtstrahlen (8), welche aus dem Wechselwirkungsbereich (W) kommend auf den Lichteinfallsbereich (51) gerichtet sind und durch den Lichteinfallsbereich (51) in das optische System (5) einfallen, mittels des Lichtführungssystems (56) weg von den Röntgenstrahlen (7), welche aus dem Bereich des Strahlengangs (27) kommend auf den Lichteinfallsbereich (51) gerichtet sind, und hin zu der Kamera (50) geführt werden, indem sie mittels eines Lichtleiters (57) des Lichtführungssystems (56) umgeleitet werden.

14. Verfahren nach Anspruch 12 oder 13
- wobei die Lichtstrahlen (8), welche aus dem Wechselwirkungsbereich (W) kommend auf den Lichteinfallsbereich (51) gerichtet sind und durch den Lichteinfallsbereich (51) in das optische System (5) einfallen, mittels des Lichtführungssystems (56) weg von den Röntgenstrahlen (7), welche aus dem Bereich des Strahlengangs (27) kommend auf den Lichteinfallsbereich (51) gerichtet sind, und hin zu der Kamera (50) geführt werden, indem sie mittels einer Lichtablenkeinheit (57) des Lichtführungssystems (56) abgelenkt werden.

15. Verfahren nach einem der Ansprüche 11 bis 14,
- wobei die Kamera (50) mittels einer Strahlenschutzkomponente des Schutzsystems (6) gegen die Röntgenstrahlung abgeschirmt wird, welche für die Lichtstrahlen (8), welche aus dem Wechselwirkungsbereich (W) kommend auf den Lichteinfallsbereich (51) gerichtet sind und durch den Lichteinfallsbereich (51) in das optische System (5) einfallen, transparent ist,
- wobei die Lichtstrahlen (8), welche aus dem Wechselwirkungsbereich (W) kommend auf den Lichteinfallsbereich (51) gerichtet sind und durch den Lichteinfallsbereich (51) in das optische System (5) einfallen, durch die Strahlenschutzkomponente des Schutzsystems (6) hindurchgeführt werden.

## Claims

1. Medical imaging device (1), having
- a beam path (27) for X-ray radiation,
- an interaction region (W), in which an examination object (14) can be arranged for an imaging examination, wherein the beam path (27) runs through the interaction region (W),
- an optical system (5) with a light incidence region (51) and a camera (50) for detecting camera image data from the interaction region (W) based on light beams (8) which are directed at the light incidence region (51) coming from the interaction region (W) and are incident into the optical system (5) through the light incidence region (51), and
- a protection system (6) for protecting the camera (50) from X-rays (7), which are directed at the light incidence region (51) coming from a region of the beam path (27),
- **characterised in that** the protection system (6) has a radiation-resistant electronic component of the camera (50), wherein the radiation-resistant electronic component of the camera (50) is an image sensor of the camera (50).

2. Medical imaging device (1) according to claim 1,
- wherein the protection system (6) has a light guiding system (56), which is designed to guide the light beams (8), which are directed at the light incidence region (51) coming from the interaction region (W) and incident into the optical system (5) through the light incidence region (51), away from the X-rays (7) which are directed at the light incidence region (51) coming from the region of the beam path (27), and towards the camera (50).

3. Medical imaging device (1) according to claim 2,
- wherein the light guiding system (56) has a light conductor (57).

4. Medical imaging device (1) according to claim 2 or 3,
- wherein the light guiding system (56) has a light deflection unit (58).

5. Medical imaging device (1) according to one of claims 2 to 4,
- wherein the light guiding system (56) is endoscopic and/or periscopic.

6. Medical imaging device (1) according to one of claims 1 to 5,
- wherein the protection system (6) has a beam protection component, which is transparent for the light beams (8) which are directed at the light incidence region (51) coming from the interaction region (W) and are incident into the optical system (5) through the light incidence region (51), and shields the camera (50) from the X-ray radiation.

7. Medical imaging device (1) according to claim 6,
- wherein the radiation protection component is produced from radiation protection glass.

8. Medical imaging device (1) according to claim 6 or 7,
- wherein the radiation protection component is a lens of the optical system (5).

9. Medical imaging device (1) according to one of claims 1 to 8,
- wherein the protection system (6) has a radiation-resistant electronic component of the camera (50).

10. Medical imaging device (1) according to one of claims 1 to 9,
- wherein the medical imaging device (1) has a gantry (20) with an opening (9),
- wherein the examination object (14) can be introduced into the opening (9) for the imaging examination,
- wherein the interaction region (W) is located in the opening (9),
- wherein the light incidence region (51) is integrated into a wall (V) of the gantry (20) which bounds the opening (9).

11. Method for detecting camera image data from an interaction region (W) of a medical imaging device (1), wherein the medical imaging device (1) has a beam path (27) for X-ray radiation, wherein the beam path (27) runs through the interaction region (W), wherein an examination object (14) can be arranged in the interaction region (W) for an imaging examination, the method comprising
- detecting (S1) the camera image data from the interaction region (W) of the medical imaging device (1) by means of a camera (50) of an optical system (5) based on light beams (8), which are directed at a light incidence region (51) of the optical system (5) coming from the interaction region (W) and are incident into the optical system (5) through the light incidence region (51), and
- protecting (S2) the camera (50) from X-rays (7), which are directed at the light incidence region (51) coming from a region of the beam path (27), wherein the medical imaging device (1) has a protection system (6) for protecting the camera (50) from the X-rays (7), which are directed at the light incidence region (51) coming from the region of the beam path (27),
- **characterised in that** the protection system (6) has a radiation-resistant electronic component of the camera (50), wherein the radiation-resistant electronic component of the camera (50) is an image sensor of the camera (50).

12. Method according to claim 11,
- wherein the light beams (8), which are directed at the light incidence region (51) coming from the interaction region (W) and are incident into the optical system (5) through the light incidence region (51), are directed by means of a light guiding system (56) away from the X-rays (7), which are directed at the light incidence region (51) coming from the region of the beam path (27) and are guided towards the camera (50).

13. Method according to claim 12,
- wherein the light beams (8), which are directed at the light incidence region (51) coming from the interaction region (W) and are incident into the optical system (5) through the light incidence region (51), are guided by means of the light guiding system (56) away from the X-rays (7), which are directed at the light incidence region (51) coming from the region of the beam path (278), and towards the camera (50), by being redirected by means of a light conductor (57) of the light guiding system (56).

14. Method according to claim 12 or 13,
- wherein the light beams (8), which are directed at the light incidence region (51) coming from the interaction region (W) and are incident into the optical system (5) through the light incidence region (51), are guided by means of the light guiding system (56) away from the X-rays (7) which are directed at the light incidence region (51) coming from the region of the beam path (27) and towards the camera (50), by being deflected by means of a light deflection unit (57) of the light guiding system (56).

15. Method according to one of claims 11 to 14,
- wherein the camera (50) is shielded against X-ray radiation by means of a radiation protection component of the protection system (6), which is transparent for the light beams (8) which are directed at the light incidence region (51) coming from the interaction region (W) and are incident into the optical system (5) through the light incidence region (51),
- wherein the light beams (8), which are directed at the light incidence region (51) coming from the interaction region (W) and are incident into the optical system (5) through the light incidence region (51), are guided through the radiation protection component of the protection system (6).

## Revendications

1. Appareil (1) d'imagerie médicale, comportant
- un trajet (27) de rayon pour un rayonnement X,
- une zone (W) d'interaction, dans laquelle on peut disposer un objet (14) à examiner pour un examen par imagerie, dans lequel le trajet (27) de rayon traverse la zone (W) d'interaction,
- un système (5) optique ayant une zone (51) d'incidence de la lumière et une caméra (50) de saisie de données d'image de caméra de la zone (W) d'interaction sur la base de rayons (8) lumineux, qui, venant de la zone (W) d'interaction, sont dirigés sur la zone (51) d'incidence de la lumière et qui arrivent dans le système (5) optique par la zone (51) d'incidence de la lumière, et
- un système (6) de protection pour la protection de la caméra (50) de rayons (7) X, qui, venant d'une zone du trajet (27) des rayons, sont dirigés sur la zone (51) d'incidence de la lumière,
- **caractérisé en ce que** le système (6) de protection a un composant électronique de la caméra (50) résistant au rayonnement, dans lequel le composant électronique de la caméra (50) résistant au rayonnement est un capteur d'image de la caméra (50).

2. Appareil (1) d'imagerie médicale suivant la revendication 1,
- dans lequel le système (6) de protection a un système (56) de conduite de la lumière, qui est agencé pour éloigner les rayons (8) lumineux, qui sont dirigés en venant de la zone (W) d'interaction sur la zone (51) d'incidence de la lumière et qui arrivent dans le système (5) optique par la zone (51) d'incidence de la lumière, des rayons (7) X, qui, venant de la zone du trajet (27) des rayons, sont dirigés sur la zone (51) d'incidence de la lumière et vers la caméra (50).

3. Appareil (1) d'imagerie médicale suivant la revendication 2,
- dans lequel le système (56) de conduite de la lumière est une fibre (57) optique.

4. Appareil (1) d'imagerie médicale suivant la revendication 2 ou 3,
- dans lequel le système (56) de conduite de la lumière a une unité (58) de déviation de la lumière.

5. Appareil (1) d'imagerie médicale suivant l'une des revendications 2 à 4,
- dans lequel le système (56) de conduite de la lumière est endoscopique et/ou périscopique.

6. Appareil (1) d'imagerie médicale suivant l'une des revendications 1 à 5,
- dans lequel le système (6) de protection a un composant de protection vis-à-vis des rayons, qui pour les rayons (8) lumineux, qui, arrivant de la zone (W) d'interaction, sont dirigés sur la zone (51) d'incidence de la lumière et arrivent dans le système (5) optique par la zone (51) d'incidence de la lumière, est transparent et protège la caméra (50) vis-à-vis du rayonnement X.

7. Appareil (1) d'imagerie médicale suivant la revendication 6,
- dans lequel le composant de protection vis-à-vis des rayons est fabriqué en un verre protégeant des rayons.

8. Appareil (1) d'imagerie médicale suivant la revendication 6 ou 7,
- dans lequel le composant de protection vis-à-vis des rayons est une lentille du système (5) optique.

9. Appareil (1) d'imagerie médicale suivant l'une des revendications 1 à 8,
- dans lequel le système (6) de protection a un composant électronique de la caméra (50) résistant au rayonnement.

10. Appareil (1) d'imagerie médicale suivant l'une des revendications 1 à 9,
- dans lequel l'appareil (1) d'imagerie médicale a un portique (20) ayant une ouverture (9),
- dans lequel l'objet (14) à examiner peut être introduit dans l'ouverture (9) pour l'examen par imagerie,
- dans lequel la zone (W) d'interaction se trouve dans l'ouverture (9),
- dans lequel la zone (51) d'incidence de la lumière est intégrée dans une paroi (V), délimitant l'ouverture (9) du portique (20).

11. Procédé de saisie de données d'image de caméra d'une zone (W) d'interaction d'un appareil (1) d'imagerie médicale, dans lequel l'appareil (1) d'imagerie médicale a un trajet (27) de rayon pour un rayonnement X, dans lequel le trajet (27) de rayon traverse la zone (W) d'interaction, dans lequel on peut disposer un objet (14) à examiner pour un examen par imagerie dans la zone (W) d'interaction, le procédé comprenant
- une saisie (S1) des données d'image de la caméra de la zone (W) d'interaction de l'appareil (1) d'imagerie médicale au moyen d'une caméra (50) d'un système (5) optique reposant sur des rayons (8) lumineux, qui sont dirigés en arrivant de la zone (W) d'interaction sur une zone (51) d'incidence de la lumière du système (5) optique et arrivent dans le système (5) optique par la zone (51) d'incidence de la lumière, et
- une protection (S2) de la caméra (50) vis-à-vis de rayons (7) X, qui sont dirigés en venant d'une zone du trajet (27) des rayons sur la zone (51) d'incidence de la lumière, dans lequel l'appareil (1) d'imagerie médicale a un système (6) de protection pour la protection de la caméra (50) de rayons (7) X, qui, en venant de la zone du trajet (27) de rayon, sont dirigés sur la zone (51) d'incidence de la lumière,
- **caractérisé en ce que** le système (6) de protection a un composant électronique de la caméra (50) résistant au rayonnement, dans lequel le composant électronique de la caméra (50) résistant au rayonnement est un capteur d'image de la caméra (50).

12. Procédé suivant la revendication 11,
- dans lequel les rayons (8) lumineux, qui, en venant de la zone (W) d'interaction, sont dirigés sur la zone (51) d'incidence de la lumière et arrivent dans le système (5) optique par la zone (51) d'incidence de la lumière, sont, au moyen d'un système (56) de conduite de la lumière, éloignés des rayons (7) X, qui,
- en venant de la zone du trajet (27) des rayons, sont dirigés sur la zone (51) d'incidence de la lumière et de la caméra (50).

13. Procédé suivant la revendication 12,
- dans lequel on éloigne les rayons (8) lumineux, qui, en venant de la zone (W) d'interaction, sont dirigés sur la zone (51) d'incidence de la lumière et arrivent dans le système (5) optique par la zone (51) d'incidence de la lumière, au moyen du système (56) de conduite de la lumière, des rayons (7) X, qui, en venant de la zone du trajet (27) des rayons, sont dirigés sur la zone (51) d'incidence de la lumière et de la caméra (50), en les déviant au moyen d'une fibre (57) optique du système (56) de conduite de la lumière.

14. Procédé suivant la revendication 12 ou 13,
- dans lequel on éloigne les rayons (8) lumineux, qui, en venant de la zone (W) d'interaction, sont dirigés sur la zone (51) d'incidence de la lumière et arrivent dans le système (5) optique par la zone (51) d'incidence de la lumière, au moyen du système (56) de conduite de la lumière, des rayons (7) X, qui, en venant de la zone du trajet (27) des rayons, sont dirigés sur la zone (51) d'incidence de la lumière et vers la caméra (50), en les déviant, au moyen d'une unité (57) de déviation de la lumière du système (56) de conduite de la lumière.

15. Procédé suivant l'une des revendications 11 à 14,
- dans lequel au moyen d'un composant de protection vis-à-vis du rayonnement du système (6) de protection, on protège la caméra (50) du rayonnement X, qui pour les rayons (8) lumineux, qui, venant de la zone (W) d'interaction, sont dirigés sur la zone (51) d'incidence de la lumière et arrivent dans le système (5) optique par la zone (51) d'incidence de la lumière, est transparent,
- dans lequel on fait passer les rayons (8) lumineux, qui, en venant de la zone (W) d'interaction, sont dirigés sur la zone (51) d'incidence de la lumière et arrivent dans le système (5) optique par la zone (51) d'incidence de la lumière, dans le composant de protection vis-à-vis des rayons du système (6) de protection.
